# EUROPÄISCHE PATENTSCHRIFT

(11) **EP 0 753 510 B1**
(45) Veröffentlichungstag und Bekanntmachung des Hinweises auf die Patenterteilung: **21.10.1998**
(21) Anmeldenummer: 96111164.8
(22) Anmeldetag: 11.07.1996
(51) Int. Cl.: C07C 401/00

(54) **Verfahren zur Herstellung von 9,10-Secocholesta-5,7,10(19)-trienen**
Method of production of 9,10-secocholesta-5,7,10(19)-trienes
Méthode de production des 9,10-sécocholesta-5,7,10(19)-triènes

(30) Priorität: 11.07.1995 CH 2042/95
(43) Veröffentlichungstag der Anmeldung: 15.01.1997
(73) Patentinhaber: Cerbios-Pharma S.A., CH-6917 Barbengo (CH)
(72) Erfinder: Schickli, Christof, Dr., 6976 Castagnola (CH); Jud, Mathias, 6904 Lugano (CH); Marazza, Fabrizio, Dr., 6986 Novaggio (CH)
(74) Vertreter: Zink-Wild, Markus Peter

(56) Entgegenhaltungen:
- EP-A- 0 402 982
- WO-A-87/00834
- CH-A- 678 853
- JOURNAL OF THE AMERICAN CHEMICAL SOCIETY, Bd. 116, Nr. 18, September 1994, DC US, Seiten 8112-8115, XP002018401 CURCI R ET AL: "Oxidation of Natural Targets by Dioxiranes. 3.1 Stereoselective Synthesis of (all-R)-Vitamin D3 Triepoxide and of Its 25-Hydroxy Derivative"
- TETRAHEDRON LETTERS, Nr. 13, März 1977, OXFORD GB, Seiten 1107-1108, XP002018402 B. L. ONISKO ET AL: "Synthesis of Potential Vitamin D Anagonists"
- JOURNAL OF ORGANIC CHEMISTRY, Bd. 57, Nr. 17, August 1992, EASTON US, Seiten 4717-4722, XP002018403 XIAOGEN YANG ET AL: "Hydrolysis and Reversible Isomerization of Humulene Epoxides II and III"
- CANADIAN JOURNAL OF CHEMISTRY, Bd. 68, Nr. 1, 1990, OTTAWA CA, Seiten 153-185, XP002018404 P. DESLONGCHAMPS ET AL: "The Total Synthesis of (+)-Ryanodol. Part III. Preparation of (+)-Anhydroryanodol from A Key Pentacyclic Intermediate"
- JOURNAL OF THE AMERICAN CHEMICAL SOCIETY, Bd. 112, Nr. 12, 1990, DC US, Seiten 4988-4989, XP002018405 K. C. NICOLAOU ET AL: "Novel Stereocontrolled Synthesis of the Nonacene Ring System of Brevetoxin A. Conformational-Reactivity Effects in Nine-Membered Rings"
- JOURNAL OF THE AMERICAN CHEMICAL SOCIETY, Bd. 94, Nr. 18, September 1972, DC US, Seiten 6538-6540, XP002018406 K. B. SHARPLESS ET AL: "Lower Valent Tungsten Halides. A New Class of Reagents for Deoxygenation of Organic Molecules"
- BULLETIN DE LA SOCIETE CHIMIQUE DE FRANCE, Nr. 3, 1965, PARIS FR, Seiten 646-649, XP002018407 N. LE BOULCH ET AL: "Epoxidations Sélectives de l'Ergocalciférol"
- PROCEEDINGS OF THE WORKSHOP ON VITAMIN D (6TH)(VITAMIN D), Bd. 6, 1985, Seiten 790-791, XP002018408 W. REICHSL ET AL: "Selective Oxidations of the Vitamin D3 Triene System"

## Beschreibung

Die vorliegende Erfindung betrifft ein Verfahzur Herstellung von 9,10-Secocholesta-5,7,10(19)-trienen.

Die vorliegende Erfindung betrifft auch ein Verfahren zur Herstellung von 5,6:7,8:10(19)-Triepoxy-9,10-secocholesta-24-enen.

Die vorliegende Erfindung betrifft ferner 5,6:7,8-Diepoxy-9,10-secocholesta-10(19)-ene und 5,6:7,8:10(19)-Triepoxy-9,10-secocholesta-24-ene.

Vitamin D₃ der Formel 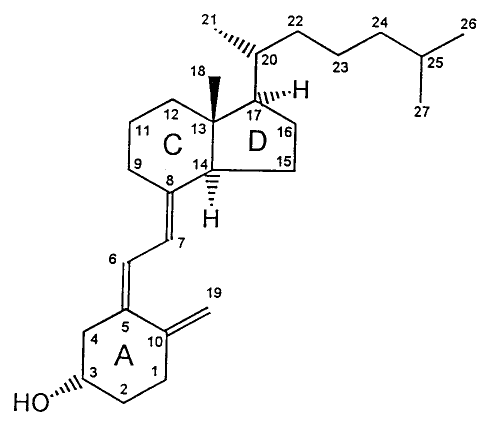 wird auch (5Z,7E)-3β-Hydroxy-9,10-secocholesta-5,7,10(19)-trien genannt.

Ueber die pharmakologische Bedeutung von Vitamin D₃ und seinen Derivaten sind zahlreiche Review Artikel erschienen, siehe z.B.:
Norman, A.W.; Bouillon, R.; Thomasset, M. (Eds), Vitamin D, A Pluripotent Steroid Hormone: Structural Studies, Molecular Endocrinology and Clinical Applications; Walter de Gruter: Berlin, New York, 1994, und hierin zitierte Referenzen.

Natürliche Metaboliten von Vitamin D₃ sind 25-Hydroxy-Vitamin D₃ und 1,25-Dihydroxy-Vitamin D₃, wobei die genannte Dihydroxyverbindung für die biologische Aktivität verantwortlich ist.

Es wurden verschiedene Methoden entwickelt, um die oben genannten Metaboliten synthetisch herzustellen. Dabei wurden folgende grundsätzlichen Wege eingeschlagen:
1. Derivatisierung von Steroiden. Siehe zum Beispiel
   - Barton, D.H.R.; Hesse, R.H.; Pechet, M.M.; Rizzardo, E.,J.Am.Chem.Soc.1973,95,2748. (A Convenient Synthesis of 1α-Hydroxy-Vitamin D₃).
   - Barton, D.H.R.; Hesse, R.H.; Pechet, M.M.; Rizzardo, E.,J.Chem.Soc.Chem.Commun. 1974,203. (Convenient Synthesis of Crystalline 1α,25-Dihydroxyvitamin D₃).
2. Konvergente Synthese mittels Kombination des derivatisierten A-Ringsystems mit dem derivatisierten C/D-Ringsystem. Siehe zum Beispiel
   - Baggiolini, E.G.; Iacobelli, J.A.; Hennessy, B.M.; Batcho, A.D.; Sereno, J.F.; Uskokovic, M.R.,J.Org. Chem. 1986,51,3098. (Stereocontrolled Total Synthesis of 1α,25-Dihydroxycholecalciferol and 1α,25-Dihydroxyergocalciferol).
3. Direkte Funktionalisierung von Vitamin D₃.

Wenn Vitamin D₃ direkt funktionalisiert werden soll, dann muss vorgängig das Trien-System geschützt werden.

So verwendete H. DeLuca gemäss CH PS 658 050 bei seiner 1α-Hydroxylierung einen Cyclovitamin D₃-methylaether als Schutzgruppe.

Die von R. Hesse beschriebene Methode gemäss EP 0 078 704 B1 verwendet ein Diels-Alder-Addukt von S0₂ mit Vitamin D.

Im Hinblick auf die Einfühgung der Hydroxygruppe in Stellung 25 haben diese beiden Methoden den Nachteil, dass die Seitenkette zuerst abgebaut und dann wieder modifiziert aufgebaut werden muss.

Eine bekannte Methode zum Schutz von Doppelbindungen besteht darin, die Doppelbindung in ein Epoxid überzuführen.

Mono-, di- und tri-epoxide von Vitamin D sind in der Literatur bekannt, sind aber weder in der Stellung 1 noch in der Stellung 25 funktionalisiert. Siehe beispielsweise J. Org. Chem. 1984, 49, 1537, und Monatsh. Chem. 1985, 116, 831 und hierin zitierte Referenzen.

R. Curci et al. beschreibt im J. Am. Chem. Soc. 1994, 116, 8112 - 8115 ein Eintopfverfahren zur regiospezifischen Hydroxylierung der Stellung 25, verbunden mit dem Schutz des Trien-Systems als Triepoxid.

Diese Methode ist ideal für die Einführung der Hydroxygruppe in Stellung 25, setzt aber voraus, dass aus dem Triepoxid selektiv das Trien-System des Vitamin D₃ regeneriert werden kann.

Solche Regenerierungsmethoden werden aber nicht beschrieben.

Die Regenerierung von nicht konjugierten Doppelbindungen aus nicht benachbarten Epoxiden mittels dem Reagenz WCl₆/n-BuLi ist in der Literatur beschrieben; siehe beispielsweise K.B. Sharpless et al., J. Am. Chem. Soc. (1972), 94, 6538; Journal of the American Chemical Society, Bd. 94, Nr. 18, 1972, Seiten 6538-6540; Journal of Organic Chemistry, Bd. 57, Nr. 17, 1992, Seiten 4717-4722; Canadian Journal of Chemistry, Bd. 68, Nr. 1, 1990, Seiten 153 - 185 und Journal of the American Chemical Society, Bd. 112, Nr. 12, 1990, Seiten 4988 - 4989.

Es ist ein Ziel der vorliegenden Erfindung, ein Verfahren zur Herstellung von in Stellung 1 und/oder in Stellung 25 hydroxylierten 9,10-Secocholesta-5,7,10(19)-trienen zur Verfügung zu stellen.

Dieses Verfahren soll die direkte oder stufenweise Regenerierung des Trien-Systems aus den Triepoxiden, beispielsweise hergestellt gemäss dem oben erwähnten Verfahren von R. Curci, beinhalten.

Dieses Verfahren soll bei einer stufenweisen Regenerierung des Trien-Systems auch noch die Möglichkeit beinhalten, in Stellung 1 eine Hydroxygruppe einzuführen.

Dieses Verfahren soll technisch einfach durchführbar sein und wenig Stufen umfassen.

Es ist ein weiteres Ziel der vorliegenden Erfindung, Derivate von Vitamin D₃ zur Verfügung zu stellen, in welchen das Trien-System als Triepoxid geschützt ist und in der Seitenkette eine solche funktionelle Gruppe aufweisen, welche weitere Modifizierungen zulassen.

Es soll auch ein Verfahren zur Herstellung dieser Derivate zur Verfügung gestellt werden.

Diese Ziele werden mit den erfindungsgemässen Verfahren und den erfindungsgemässen Verbindungen erreicht.

Das erste erfindungsgemässe Verfahren zur Herstellung von 9,10-Secocholesta-5,7,10(19)-trienen der Formel I 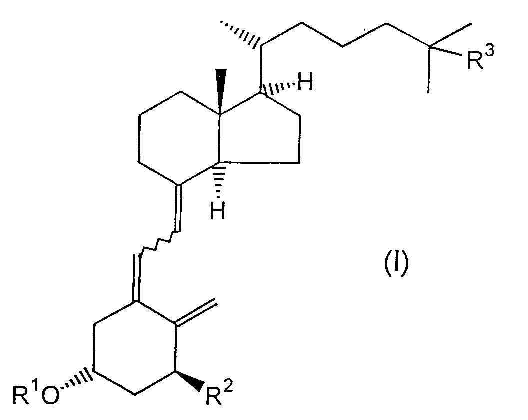 worin
R¹ Wasserstoff oder eine Schutzgruppe für die Hydroxyfunktion, an welche R¹ gebunden ist, darstellt,
R² Wasserstoff oder OR¹ ist, worin R¹ weiter oben definiert ist,
R³ Wasserstoff oder OR¹ ist, worin R¹ weiter oben definiert ist,
   wobei die Gruppen R¹, R² und R³ unabhängig voneinander ausgewählt werden können,
   ist dadurch gekennzeichnet, dass man
   - in einem ersten Schritt ein Uebergangsmetallhalogenid der Formel II

      M(z)X_{z} (II)

      worin
      - M(z): W(VI), Nb(V), Mo(V), Ti(IV), Ti(III) oder Fe (III) ist,
      - X: Chlorid, Bromid oder Iodid ist,
      - z: eine ganze Zahl von 3 bis 6 bedeutet,
      mit 1 bis z Aequivalenten einer Alkylmetallverbindung oder einem Metallhydrid zu einem entsprechenden niedervalenten Metallreagenz unter einer Inertgasatmosphäre in einem wasserfreien Aether oder Kohlenwasserstoff reduziert,
   - in einem zweiten Schritt eine Verbindung der Formel III 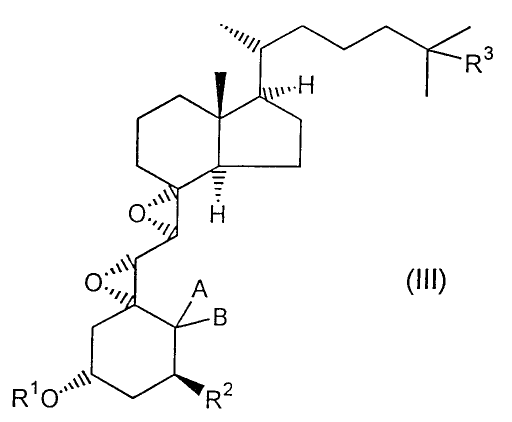 worin
      - R¹, R², R³: weiter oben definiert sind,
      - A und B: zusammen genommen entweder ein Epoxid der Formel -(O-CH₂)- oder eine exocyclische Doppelbindung der Formel =CH₂ bedeuten,
      gelöst in einem oben genannten Lösungsmittel, zum im ersten Schritt hergestellten niedervalenten Metallreagenz hinzugibt und reagieren lässt,
   - in einem dritten Schritt das so erhaltene Reaktionsprodukt aufarbeitet und das Produkt der Formel I gewinnt.

Das zweite erfindungsgemässe Verfahren zur Herstellung von 5,6:7,8:10(19)-Triepoxy-9,10-secocholesta-24-enen der Formel V 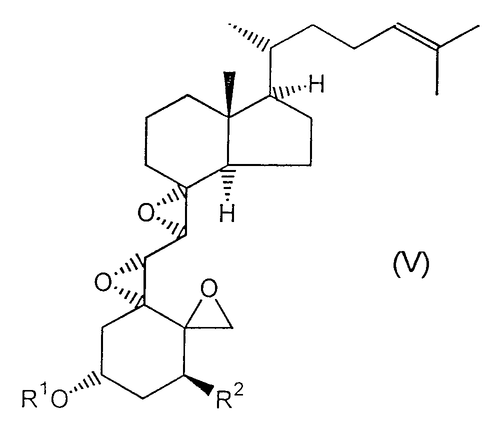 worin
R¹ Wasserstoff oder eine Schutzgruppe für die Hydroxyfunktion, an welche R¹ gebunden ist, darstellt,
R² Wasserstoff oder OR¹ ist, worin R¹ weiter oben definiert ist,
   wobei die Gruppen R¹ und R² unabhängig voneinander ausgewählt werden können,
   ist dadurch gekennzeichnet, dass man eine Verbindung der Formel VI 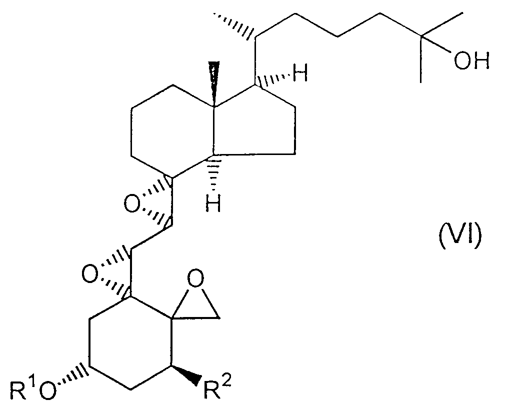 worin
R¹ und R² weiter oben definiert sind, aber wobei R¹ nicht Wasserstoff sein darf,
   mit Phosphoroxychlorid in Pyridin bei einer Temperatur von 0°C bis 30°C unter Wasserausschluss zur Reaktion bringt, das so erhaltene Reaktionsprodukt, mit oder ohne Entfernung von Schutzgruppen R¹, aufarbeitet und das Produkt der Formel V gewinnt.

Die erfindungsgemässen Verbindungen sind 5,6:7,8-Diepoxy-9,10-secocholesta-10(19)-ene der Formel IV 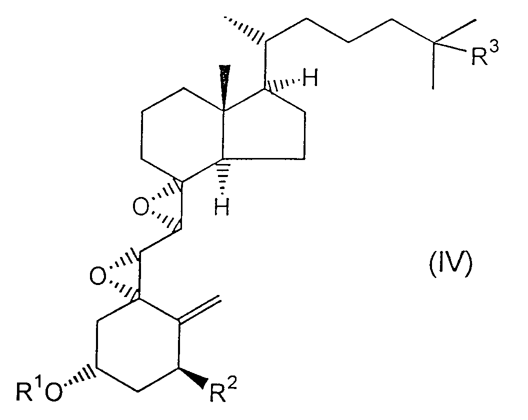 worin
R¹ Wasserstoff oder eine Schutzgruppe für die Hydroxyfunktion, an welche R¹ gebunden ist, darstellt,
R² Wasserstoff oder OR¹ ist, worin R¹ weiter oben definiert ist,
R³ Wasserstoff oder OR¹ ist, worin R¹ weiter oben definiert ist,
   wobei die Gruppen R¹, R² und R³ unabhängig voneinander ausgewählt werden können, wobei aber R² und R³ nicht gleichzeitig Wasserstoff bedeuten dürfen,
   und
   5,6:7,8:10(19)-Triepoxy-9,10-secocholesta-24-ene der Formel V 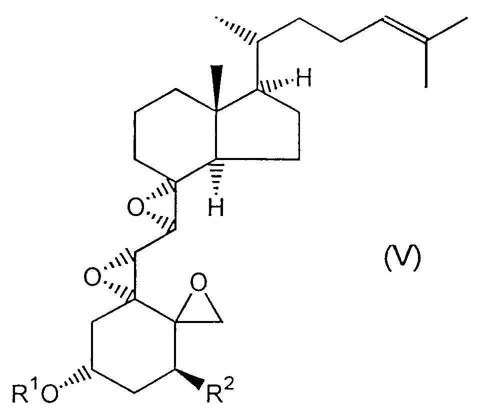 worin
R¹ Wasserstoff oder eine Schutzgruppe für die Hydroxyfunktion, an welche R¹ gebunden ist, darstellt,
R² Wasserstoff oder OR¹ ist, worin R¹ weiter oben definiert ist,
   wobei die Gruppen R¹ und R² unabhängig voneinander ausgewählt werden können.

Bevorzugte Ausführungsformen dieser Erfindungsgegenstände sind in den abhängigen Ansprüchen definiert.

Nachfolgend werden bevorzugte Ausführungsformen der Erfindungsgegenstände beschrieben.

Dabei werden Ausführungsformen, wie sie in den abhängigen Ansprüchen definiert sind, normalerweise nicht wiederholt.

Die Ausgangsmaterialien der Formel III werden vorzugsweise gemäss dem oben erwähnten Artikel von R. Curci hergestellt.

In bevorzugten Triepoxiden der Formel III ist R¹ Acetyl oder Benzoyl, R² ist Wasserstoff und R³ ist Hydroxyl.

Für die direkte Regenerierung des Triensystems wird die 25-Hydroxygruppe acetyliert oder mit Trimethylsilan geschützt.

Diese acetylierte oder trimethylsilylierte Verbindung wird danach mit dem WCl₆ / n-Butyllithium-System in Tetrahydrofuran umgesetzt.

Bei dieser direkten Regenerierung des Triensystems wird ein 5Z:5E Isomerenverhältnis von 1:9 erhalten.

Bei der stufenweisen Regenerierung des Triensystems wird nach der Regenerierung der 10(19)-Doppelbindung die 25-Hydroxygruppe acetyliert.

Auch diese acetylierte Verbindung wird danach mit dem WCl₆ / n-Butyllithium-System in Tetrahydrofuran umgesetzt.

Bei dieser stufenweisen Regenerierung des Triensystems wird ein 5Z:5E Isomerenverhältnis von 3:1 erhalten.

Das unerwünschte 5E-Isomer der Formel I kann entweder chromatographisch oder mittels der Maleinsäureanhydrid-Methode gemäss US PS 4 554 106 abgetrennt werden.

Im Formelschema sind die Reaktionswege zusammengefasst.

### Formelschema

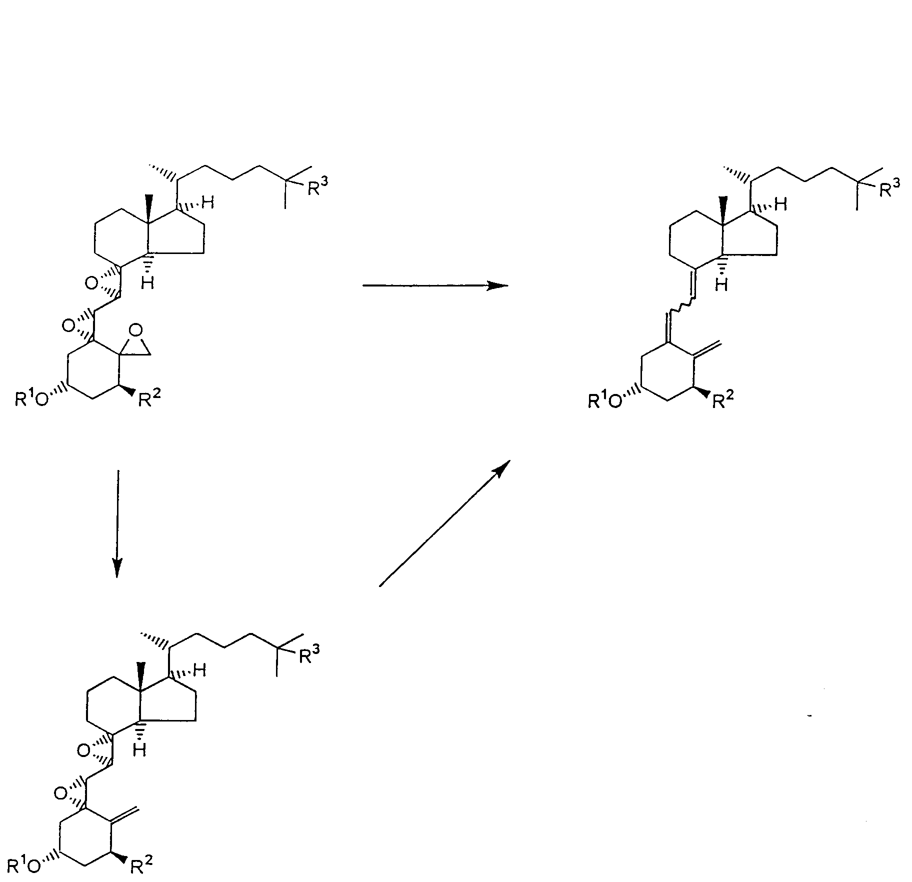

Die Wasserabspaltung in der Seitenkette der Verbindungen der Formel VI erfolgt in Analogie zu jener im Artikel von L. Onisko et.al. Biochem. J. (1979) 182, 1 - 9 sowie B.L. Onisko et. al., Tetrahedron Letters, Nr. 13, März 1977, Seiten 1107 - 1108.

In bevorzugten Ausgangsmaterialien der Formel VI ist R¹ Acetyl oder Benzoyl und R² ist Wasserstoff.

Das Isomerenverhältnis von 24-en : 25-en beträgt 2:1.

Die so in Stellung 24 eingeführte Doppelbindung erlaubt weitere Modifizierungen in der Seitenkette.

Die nachfolgenden Beispiele illustrieren die vorliegende Erfindung.

### Beispiel 1

Zu einer Lösung von 2 g (4.1 mmol) 3β-Acetoxy-25-hydroxy-5,6:7,8:10(19)-triepoxy-9,10-secocholestan und 108 mg (0.41 mmol) 18-Crown-6 in 65 ml trockenem Tetrahydrofuran fügte man 5.88 g (41 mmol) KSeCN zu.

Diese Suspension erhitzte man für 72 Stunden unter Rückfluss. Danach wurde die Suspension mit 200 ml Diethylether verdünnt und 2 x mit je 150 ml H₂0, 1 x mit 150 ml 1N HCl und 1 x mit 150 ml gesättigter NaCl-Lösung extrahiert.

Die organische Phase wurde über MgSO₄ getrocknet, und danach wurde das Lösungsmittel abdestilliert.

Die Flash Chromatographie (Kieselgel; Petrolether/Ethylacetat 5:2) ergab 920 mg (48 %) reines 3β-Acetoxy-25-hydroxy-5,6:7,8-diepoxy-9,10-secocholest-10(19)-en.
¹H-NMR (90 MHz, CDCl₃, TMS): 0.63 (s, 3H, H-C(18)), 1.17 (s, 6H, H-C(26,27)), 2.00 (s, 3H, H-acetyl), 2.80 (AB, 2H, H-C(6,7)), 4.8 (m, 1H, H-C(3)), 4.94 (s, br., 1H, Hₜᵣₐₙₛ-C(19)), 5.07 (s, br. 1H, H_{cis}-C(19)).

### Beispiel 2

Man kühlte eine Lösung von 400 mg (0.84 mmol) 3β-Acetoxy-25-hydroxy-5,6:7,8-diepoxy-9,10-secocholest-10(19)-en und 0,31 g (2.53 mmol) p-Dimethylaminopyridin in 6 ml trockenem CH₂Cl₂ auf eine Temperatur von 0°C ab und tropfte langsam 0.24 ml (2.53 mmol) Essigsäureanhydrid zu.

Innerhalb von 20 Stunden liess man die Reaktion auf Raumtemperatur erwärmen, verdünnte mit 30 ml CH₂Cl₂ und extrahierte mit je 30 ml gesättigter NaHCO₃-Lösung, 1N HCl und gesättigter NaCl-Lösung.

Die organische Phase trocknete man über MgSO₄, verdampfte das Lösungsmittel am Rotationsverdampfer und trocknete unter Vakuum.

Man erhielt 356 mg (82 %) 3β,25-Diacetoxy-5,6:7,8-diepoxy-9,10-secocholest-10(19)-en als gelben, klebrigen Schaum.
¹H-NMR (90 MHz, CDCl₃, TMS): 0.63 (s, 3H, H-C(18)), 1.17 (s, 6H, H-C(26,27)), 1.91 (s, 3H, H-acetyl (25)), 2.00 (s, 3H, H-acetyl(3)), 2.80 (AB, 2H, H-C(6, 7)), 4.8 (m, 1H, H-C(3)), 4.94 (s, br., 1H, Hₜᵣₐₙₛ-C(19)), 5.07 (s, br. 1H, H_{cis}-C(19)).

### Beispiel 3

Man suspendierte 1.07 g (2.71 mmol) WCl₆ in 12 ml trockenem Tetrahydrofuran und kühlte auf eine Temperatur von -65°C ab. Langsam tropfte man 5.2 ml (8.13 mmol) 1.6 M n-BuLi zu und liess innerhalb von 30 Minuten auf Raumtemperatur aufwärmen.

Danach kühlte man wieder auf eine Temperatur von -55°C ab und tropfte 200 mg (0.38 mmol) 3β,25-Diacetoxy-5,6:7,8-diepoxy-9,10-secocholest-10(19)-en in 4 ml trockenem Tetrahydrofuran zu.

Man erwärmte das Reaktionsgemisch wieder auf Raumtemperatur und rührte 2 Stunden nach.

Dann nahm man das Gemisch in 100 ml Diethylether auf und extrahierte 2 x mit je 200 ml einer Lösung von 1.5 M K/Na-tartrat in 2N NaOH und 1 x mit 150 ml gesättigter NaCl-Lösung.

Die organische Phase trocknete man über MgSO₄, verdampfte das Lösungsmittel am Rotationsverdampfer und trocknete unter Vakuum.

Man erhielt so 190 mg (100%) eines 3:1 5Z/5E Gemisches von 7E-3β,25-Diacetoxy-9,10-secocholesta-5,7,10(19)-trien.

Dieses Gemisch war an den vinylischen Protonen des 5,7-Dien-Systems im ¹H-NMR unterscheidbar.

### Beispiel 4

Man löste 500 mg (0,9 mmol) 3β-Benzoyloxy-25-hydroxy-5,6:7,8:10(19)-triepoxy-9,10-secocholestan in 35 ml absolutem Pyridin.

Dieses Gemisch wurde auf eine Temperatur von 0°C abgekühlt.

Danach tropfte man 2,06 ml (22,5 mmol) Phosphoroxychlorid zu. Man entfernte das Eisbad, rührte eine Stunde bei Raumtemperatur, und kühlte wieder auf 0°C ab.

Dann tropfte man 50 ml Wasser hinzu und verdünnte mit 100 ml Hexan.

Danach extrahierte man die organische Phase 4 x mit je 100 ml 1N HCl, 1 x mit 100 ml 5% NaHCO₃-Lösung und 1 x mit 100 ml gesättigter NaCl-Lösung.

Die organische Phase trocknete man über MgSO₄ und verdampfte das Lösungsmittel am Rotationsverdampfer.

Die Flash Chromatographie (Kieselgel; Petrolether/Ethylacetat 7:1) ergab 98 mg eines 2:1 Gemisches von 24-en : 25-en von 3β-Benzoyloxy-5,6:7,8:10 (19)-triepoxy-9,10-secocholestan.
¹H-NMR (90 MHz, CDCl₃, TMS) von 24-en: 3.45 (d, 1H, H-C(6 oder 7)), 5.0 (m, br., 1H, H-C(24)), 5.02 (m, 1H, H-C(3)), 7.1-7.5 (m, 3H, arom. H), 7.8-8.0 (m, 2H, arom. H).

### Beispiel 5

Man suspendierte 983 mg (2.48 mmol) WCl₆ in 12 ml trockenem Tetrahydrofuran und kühlte auf eine Temperatur von -65°C ab.

Langsam tropfte man 4.65 ml (7.44 mmol) 1.6 M n-BuLi zu und liess innerhalb von 30 Minuten auf Raumtemperatur aufwärmen.

Danach kühlte man wieder auf eine Temperatur von -55°C ab und tropfte 200 mg (0.35 mmol) 3β-Acetoxy-5,6:7,8-diepoxy-25-trimethylsilyloxy-9,10-secocholest-10(19)-en in 4 ml trockenem Tetrahydrofuran zu.

Man erwärmte das Reaktionsgemisch wieder auf Raumtemperatur und rührte 2 Stunden nach.

Dann nahm man das Gemisch in 100 ml Diethylether auf und extrahierte 2x mit je 150 ml einer Lösung von 1.5 M K/Na-tartrat in 2N NaOH und 1x mit 100 ml gesättigter NaCl-Lösung.

Die organische Phase trocknete man über MgSO₄, zog das Lösungsmittel am Rotationsverdampfer ab und trocknete unter Vakuum.

Man erhielt so 180 mg (100 %) eines 1:9 Gemisches von 5-cis/trans 7E-3β-Acetoxy-25-trimethylsilyloxy-9,10-secocholesta-5,7,10(19)-trien, unterscheidbar an den vinylischen Protonen des 5,7-Dien-Systems.

## Patentansprüche

1. Verfahren zur Herstellung von 9,10-Secocholesta-5,7,10(19)-trienen der Formel I 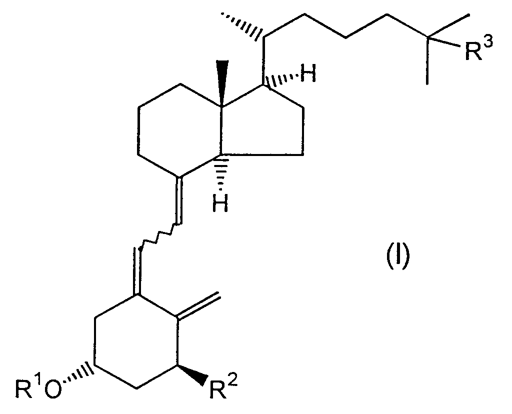 worin
R¹ Wasserstoff oder eine Schutzgruppe für die Hydroxyfunktion, an welche R¹ gebunden ist, darstellt,
R² Wasserstoff oder OR¹ ist, worin R¹ weiter oben definiert ist,
R³ Wasserstoff oder OR¹ ist, worin R¹ weiter oben definiert ist,
wobei die Gruppen R¹, R² und R³ unabhängig voneinander ausgewählt werden können,
dadurch gekennzeichnet, dass man
- in einem ersten Schritt ein Uebergangsmetallhalogenid der Formel II
M(z)X_{z} (II)
worin
M(z) W(VI), Nb(V), Mo(V), Ti(IV), Ti(III) oder Fe (III) ist,
X Chlorid, Bromid oder Iodid ist,
z eine ganze Zahl von 3 bis 6 bedeutet,
mit 1 bis z Aequivalenten einer Alkylmetallverbindung oder einem Metallhydrid zu einem entsprechenden niedervalenten Metallreagenz unter einer Inertgasatmosphäre in einem wasserfreien Aether oder Kohlenwasserstoff reduziert,
- in einem zweiten Schritt eine Verbindung der Formel III 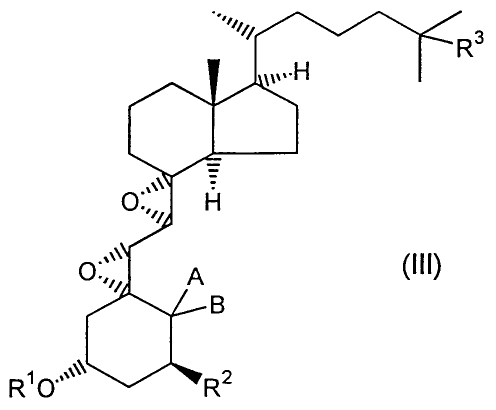 worin
R¹, R², R³ weiter oben definiert sind,
A und B zusammen genommen entweder ein Epoxid der Formel -(O-CH₂)- oder eine exocyclische Doppelbindung der Formel =CH₂ bedeuten,
gelöst in einem oben genannten Lösungsmittel, zum im ersten Schritt hergestellten niedervalenten Metallreagenz hinzugibt und reagieren lässt,
- in einem dritten Schritt das so erhaltene Reaktionsprodukt aufarbeitet und das Produkt der Formel I gewinnt.

2. Verfahren nach Anspruch 1, dadurch gekennzeichnet, dass das im ersten Schritt erwähnte Uebergangsmetallhalogenid ein Chlorid ist, insbesondere WCl₆.

3. Verfahren nach einem der Ansprüche 1 bis 2, dadurch gekennzeichnet, dass das im ersten Schritt erwähnte Reduktionsmittel n-Butyl-lithium oder Lithiumaluminiumhydrid ist.

4. Verfahren nach einem der Ansprüche 1 bis 3, dadurch gekennzeichnet, dass man pro Mol WCl₆ von 2 bis 4 Reduktionsaeguivalente einsetzt.

5. Verfahren nach einem der Ansprüche 1 bis 4, dadurch gekennzeichnet, dass man pro Mol der Verbindung der Formel III von 2 bis 9 Mol-Aequivalente an niedervalentem Metallreaganz einsetzt.

6. Verfahren nach einem der Ansprüche 1 bis 5, dadurch gekennzeichnet, dass das Lösungsmittel Diethylaether, Dioxan, Tetrahydrofuran, Dimethoxyethan, Hexan, Benzol oder Toluol ist, wobei Tetrahydrofuran bevorzugt ist.

7. Verfahren nach einem der Ansprüche 1 bis 6, dadurch gekennzeichnet, dass man das niedervalente Metallreagenz im Temperaturbereich von -78°C bis Raumtemperatur herstellt.

8. Verfahren nach einem der Ansprüche 1 bis 7, dadurch gekennzeichnet, dass die Reaktion zwischen dem niedervalenten Metallreagenz und der Verbindung der Formel III im Temperaturbereich von -78°C bis Rückflusstemperatur erfolgt.

9. Verfahren nach einem der Ansprüche 1 bis 8, dadurch gekennzeichnet, dass man zum hergestellten niedervalenten Metallreagenz vor der Hinzugabe der Verbindung der Formel III noch 2 bis 9 Aequivalente an Metalljodid hinzugibt, vorzugsweise Alkalimetalljodide, insbesondere Lithiumjodid.

10. Verfahren nach einem der Ansprüche 1 bis 9, dadurch gekennzeichnet, dass die Schutzgruppe für die Hydroxyfunktion Acetat, Benzoat, Trimethylsilyl oder tert.-Butyl-dimethyl-silyl ist.

11. Verfahren nach einem der Ansprüche 1 bis 10, dadurch gekennzeichnet, dass man eine Verbindung der Formel III, worin A und B zusammen genommen ein Epoxid der Formel -(0-CH₂)- bedeuten, mit entweder
I.) KSeCN in entweder
I.a.) einem Alkohol, insbesondere Methanol, oder
I.b.) einem Aether, insbesondere Diethylaether, Dioxan, Tetrahydrofuran, Dimethoxyethan, in Gegenwart eines Kronenaethers, insbesondere 18-Crown-6, oder
II.) (R⁴O)₂P(O)-TeLi in einem oben genannten Aether oder in R⁴OH, worin R⁴ eine Alkylgruppe bedeutet, insbesondere Ethyl oder Methyl,
unter Wasserausschluss und unter einer Inertgasatmosphäre zur Reaktion bringt, vorzugsweise unter Rückfluss, das so erhaltene Reaktionsprodukt aufarbeitet und das Produkt der Formel III, worin A und B zusammen genommen eine exocyclische Doppelbindung der Formel =CH₂ bedeuten, gewinnt.

12. Verfahren nach Anspruch 11, dadurch gekennzeichnet, dass man pro Mol der Verbindung der Formel III, worin A und B zusammen genommen ein Epoxid der Formel -(O-CH₂)- bedeuten, von 1 bis 10 Mol-Aequivalente an genannten Reagenzien einsetzt.

13. Verfahren nach einem der Ansprüche 1 bis 12, dadurch gekennzeichnet, dass man eine Verbindung der Formel III, worin
R¹ eine Schutzgruppe für die Hydroxyfunktion, an welche R¹ gebunden ist, darstellt,
R² Wasserstoff ist,
R³ in Anspruch 1 definiert ist,
A und B zusammen genommen eine exocyclische Doppelbindung der Formel =CH₂ bedeuten,
mit Selendioxid und einem Cooxidationsmittel, vorzugsweise tert.-Butyl-hydroperoxid oder Pyridinium-N-oxid, in einem Lösungsmittel, beispielsweise einem Aether, wie etwa Dioxan, Tetrahydrofuran, Dimethoxyethan, einem Kohlenwasserstoff, wie etwa Benzol, Toluol, bei einer Temperatur von mehr als 50°C zur Reaktion bringt, das so erhaltene Reaktionsprodukt aufarbeitet und das Produkt der Formel III, worin R¹, R³ sowie A und B weiter oben definiert sind, und R² eine Hydroxygruppe bedeutet, gewinnt, und gegebenenfalls diese Hydroxygruppe schützt.

14. 5,6:7,8-Diepoxy-9,10-secocholesta-10(19)-ene der Formel IV 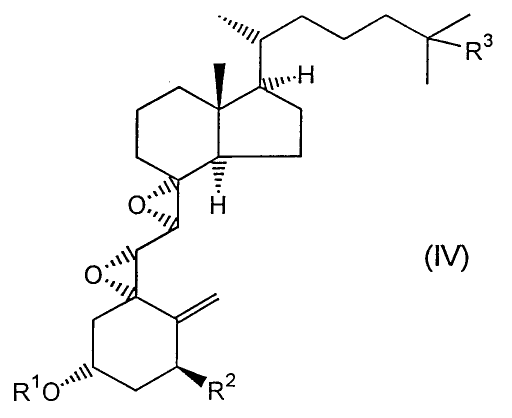 worin
R¹ Wasserstoff oder eine Schutzgruppe für die Hydroxyfunktion, an welche R¹ gebunden ist, darstellt,
R² Wasserstoff oder OR¹ ist, worin R¹ weiter oben definiert ist,
R³ Wasserstoff oder OR¹ ist, worin R¹ weiter oben definiert ist,
wobei die Gruppen R¹, R² und R³ unabhängig voneinander ausgewählt werden können, wobei aber R² und R³ nicht gleichzeitig Wasserstoff bedeuten dürfen.

15. Verfahren zur Herstellung von 5,6:7,8:10(19)-Triepoxy-9,10-secocholesta-24-enen der Formel V 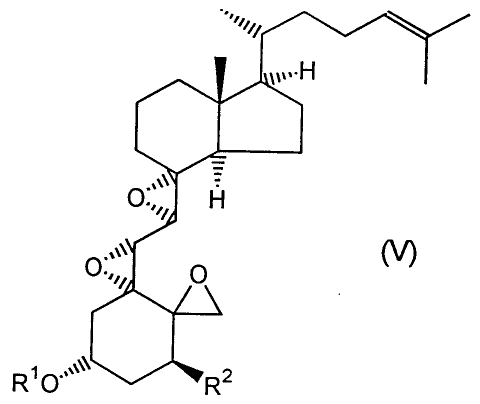 worin
R¹ Wasserstoff oder eine Schutzgruppe für die Hydroxyfunktion, an welche R¹ gebunden ist, darstellt,
R² Wasserstoff oder OR¹ ist, worin R¹ weiter oben definiert ist,
wobei die Gruppen R¹ und R² unabhängig voneinander ausgewählt werden können,
dadurch gekennzeichnet, dass man eine Verbindung der Formel VI 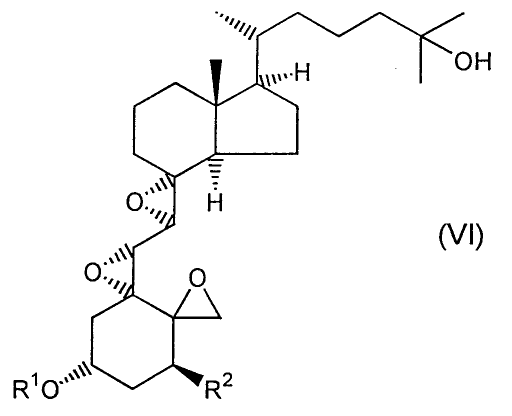 worin
R¹ und R² weiter oben definiert sind, aber wobei R¹ nicht Wasserstoff sein darf,
mit Phosphoroxychlorid in Pyridin bei einer Temperatur von 0°C bis 30°C unter Wasserausschluss zur Reaktion bringt, das so erhaltene Reaktionsprodukt, mit oder ohne Entfernung von Schutzgruppen R¹, aufarbeitet, und das Produkt der Formel V gewinnt.

16. 5,6:7,8:10(19)-Triepoxy-9,10-secocholesta-24-ene der Formel V 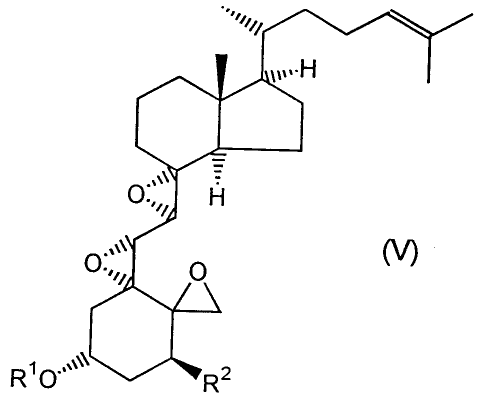 worin
R¹ Wasserstoff oder eine Schutzgruppe für die Hydroxyfunktion, an welche R¹ gebunden ist, darstellt,
R² Wasserstoff oder OR¹ ist, worin R¹ weiter oben definiert ist,
wobei die Gruppen R¹ und R² unabhängig voneinander ausgewählt werden können.

## Claims

1. A process for the preparation of 9,10-secocholesta-5,7,10(19)-trienes of the formula I 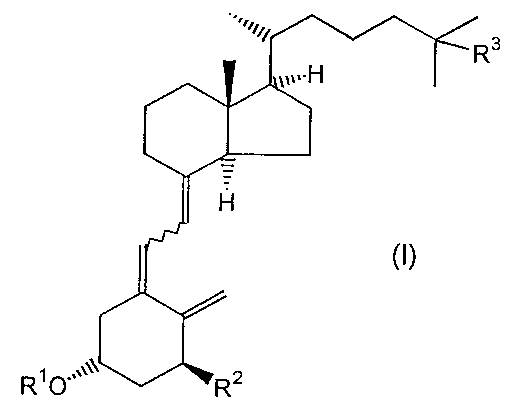 wherein
R¹ is hydrogen or a protecting group for the hydroxy function, to which R¹ is bound,
R² is hydrogen or OR¹, wherein R¹ is defined above,
R³ is hydrogen or OR¹, wherein R¹ is defined above,
whereby the groups R¹, R², and R³ may be selected independently from each other,
characterized in that
- in a first step a transition metal halide of the formula II
M(z)X_{z} (II)
wherein
M(z) is W(VI), Nb(V), Mo(V), Ti(IV), Ti(III) or Fe (III),
X is chloride, bromide or iodide,
z is an integer from 3 to 6,
is reduced with 1 to z equivalents of an alkyl metal compound or a metal hydride to a corresponding low-valent metal reagent under an inert gas atmosphere in a waterfree ether or a hydrocarbon,
- in a second step a compound of the formula III 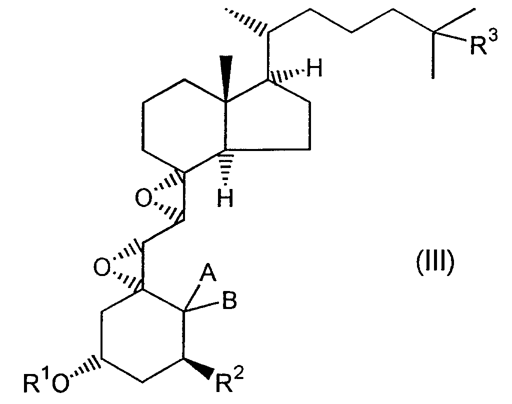 wherein
R¹, R², R³ are defined above,
A and B taken together are either an epoxide of the formula -(0-CH₂)-or an exocyclic double bond of the formula =CH₂,
dissolved in an above mentioned solvent, is added to the in the first step prepared low-valent metal reagent and is allowed to react,
- in a third step the so obtained reaction product is worked up and the product of formula I is obtained.

2. The process according to claim 1, characterized in that the in the first step mentioned transition metal halide is a chloride, especially WCl₆.

3. The process according to one of claims 1 to 2, characterized in that the in the first step mentioned reduction agent is n-butyl-lithium or lithium aluminum hydride.

4. The process according to one of claims 1 to 3, characterized in that per mole of WCl₆ from 2 to 4 reduction equivalents are used.

5. The process according to one of claims 1 to 4, characterized in that per mole of the compound of formula III from 2 to 9 mole-equivalents of low-valent metal reagent are used.

6. The process according to one of claims 1 to 5, characterized in that the solvent is diethyl ether, dioxane, tetrahydrofuran, dimethoxy ethane, hexane, benzene or toluene, whereby tetrahydrofuran is preferred.

7. The process according to one of claims 1 to 6, characterized in that the low-valent metal reagent is prepared in the temperature range from -78°C to room temperature.

8. The process according to one of claims 1 to 7, characterized in that the reaction between the low-valent metal reagent and the compound of formula III is realized in the temperature range from -78°C to reflux temperature.

9. The process according to one of claims 1 to 8, characterized in that to the prepared low-valent metal reagent are added before the addition of the compound of formula III from 2 to 9 equivalents of metal jodide, preferably alkali metal iodides, especially lithium iodide.

10. The process according to one of claims 1 to 9, characterized in that the protecting group for the hydroxy function is acetate, benzoate, trimethylsilyl or tert.-butyl-dimethyl-silyl.

11. The process according to one of claims 1 to 10, characterized in that a compound of formula III, wherein A and B taken together are an epoxide of the formula -(0-CH₂)-, is reacted under the exclusion of water and under an inert gas atmosphere, preferably under reflux, with either
I.) KSeCN in either
I.a.) an alcohol, especially methanol, or
I.b.) an ether, especially diethyl ether, dioxane, tetrahydrofuran, dimethoxy ethane, in the presence of a crown ether, especially 18-crown-6, or
II.) (R⁴O)₂P(O)-TeLi in an above mentioned ether or in R⁴OH, wherein R⁴ is an alkyl group, especially ethyl or methyl,
the so obtained reaction product is worked up, and the product of formula III, wherein A and B taken together are an exocyclic double bond of the formula =CH₂, is obtained.

12. The process according to claim 11, characterized in that per mole of the compound of formula III, wherein A and B taken together are an epoxide of the formula -(O-CH₂)-, are used from 1 to 10 mole-equivalents of said reagents.

13. The process according to one of claims 1 to 12, characterized in that a compound of formula III, wherein
R¹ is a protecting group for the hydroxy function, to which R¹ is bound,
R² is hydrogen,
R³ is defined in claim 1,
A and B taken together are an exocyclic double bond of the formula =CH₂,
is reacted at a temperature of more than 50°C with selenium dioxide and a cooxidation agent, preferably tert.-butyl-hydroperoxide or pyridinium-N-oxide, in a solvent, for example an ether, such as dioxane, tetrahydrofuran, dimethoxy ethane, a hydrocarbon, such as benzene, toluene, the so obtained reaction product is worked up and the product of formula III, wherein R¹, R³, as well as A and B are defined above, and R² is a hydroxy group, is obtained and occasionally this hydroxy group is protected.

14. 5,6:7,8-Diepoxy-9,10-secocholesta-10(19)-enes of the formula IV 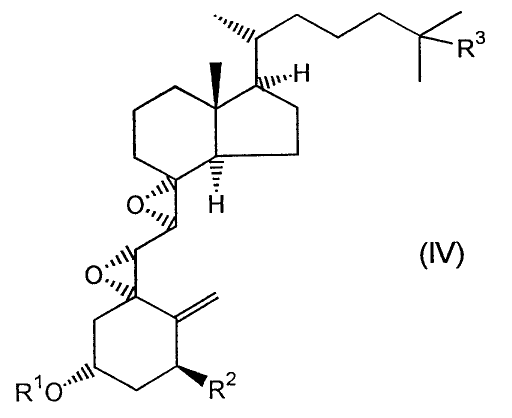 wherein
R¹ is hydrogen or a protecting group for the hydroxy function, to which R¹ is bound,
R² is hydrogen or OR¹, wherein R¹ is defined above,
R³ is hydrogen or OR¹, wherein R¹ is defined above,
whereby the groups R¹, R², and R³ may be selected independently from each other, but whereby R² and R³ may not be hydrogen at the same time.

15. A process for the preparation of 5,6:7,8:10(19)-triepoxy-9,10-secocholesta-24-enes of the formula V 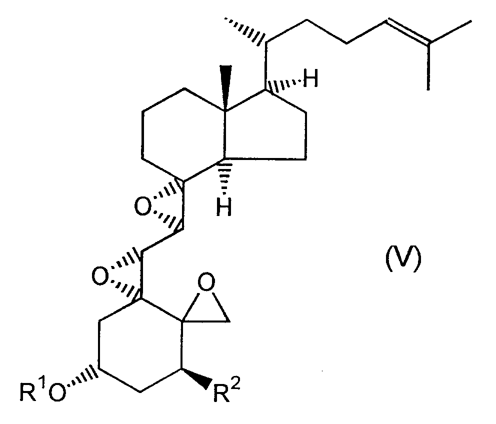 wherein
R¹ is hydrogen or a protecting group for the hydroxy function, to which R¹ is bound,
R² is hydrogen or OR¹, wherein R¹ is defined above,
whereby the groups R¹ and R² may be selected independently from each other,
characterized in that a compound of formula VI 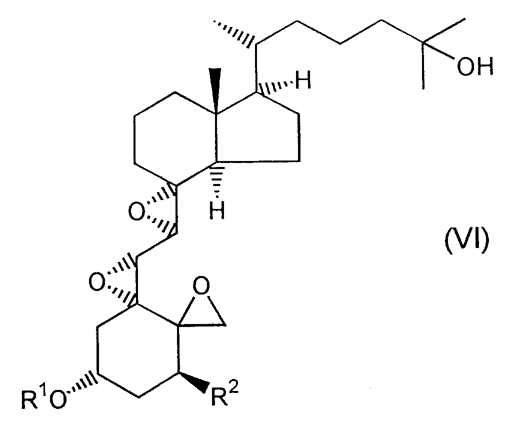 wherein
R¹ and R² are defined above, but whereby R¹ may not be hydrogen,
is reacted with a phosphorus oxychloride in pyridine at a temperature from 0°C to 30°C under the exclusion of water, the so obtained reaction product, with or without the removal of protecting groups R¹, is worked up, and the product of formula V is obtained.
16. 5,6:7,8:10(19)-Triepoxy-9,10-secocholesta-24-enes of the formula V 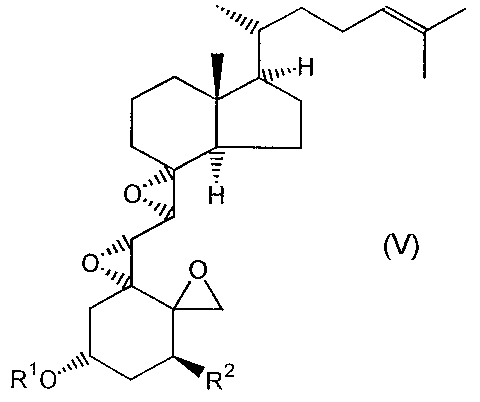 wherein
R¹ is hydrogen or a protecting group for the hydroxy function, to which R¹ is bound,
R² is hydrogen or OR¹, wherein R¹ is defined above,
whereby the groups R¹ and R² may be selected independently from each other.

## Revendications

1. Procédé pour la préparation de 9,10-sécocholesta-5,7,10(19)-triènes de formule I 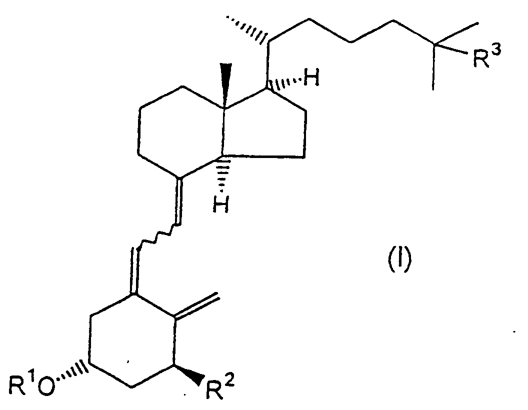 dans laquelle
R¹ représente un atome d'hydrogène ou un groupe protecteur pour la fonction hydroxyle, auquel R¹ est lié,
R² représente un atome d'hydrogène ou un groupe OR¹ où R¹ est tel que défini ci-dessus,
R³ représente un atome d'hydrogène ou un groupe OR¹ où R¹ est tel que défini ci-dessus,
les groupes R¹, R² et R³ pouvant être sélectionnés indépendamment l'un de l'autre,
caractérisé en ce que
- dans une première étape, on réduit un halogénure de métal transitoire de formule II
M(z)X_{z} (II)
dans laquelle
M(z) représente W(VI), Nb(V), Mo(V), Ti(IV), Ti(III) ou Fe(III),
X représente un chlorure, un bromure ou un iodure,
z représente un nombre entier de 3 à 6,
avec de 1 à z équivalents d'un composé alkylmétallique ou d'un hydrure métallique pour obtenir un réactif métallique de faible valence correspondant, sous l'atmosphère d'un gaz inerte, dans un éther ou dans un hydrocarbure anhydre,
- dans une deuxième étape, on dissout un composé de formule III 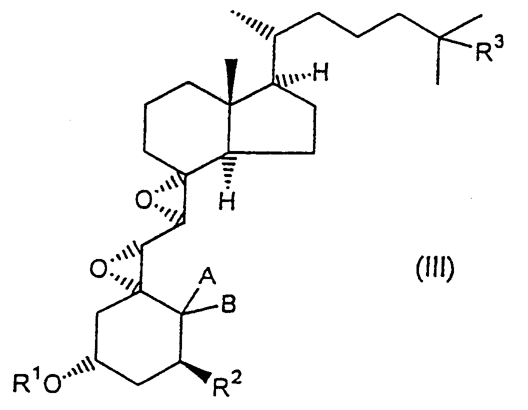
dans laquelle
R¹, R², R³ sont tels que définis ci-dessus,
A et B pris ensemble représentent, soit un époxyde de formule -(O-CH₂)-, soit une liaison double exocyclique répondant à la formule =CH₂,
dans un solvant mentionné ci-dessus, on l'ajoute au réactif métallique de faible valence préparé à la première étape et on le laisse réagir,
- dans une troisième étape, on traite le produit réactionnel ainsi obtenu et on obtient le produit de formule I.

2. Procédé selon la revendication 1, caractérisé en ce que l'halogénure de métal transitoire mentionné dans la première étape est un chlorure, en particulier WCl₆.

3. Procédé selon l'une quelconque des revendications 1 à 2, caractérisé en ce que l'agent de réduction mentionné dans la première étape est l'hydrure de n-butyllithium ou l'hydrure de lithium-aluminium.

4. Procédé selon l'une quelconque des revendications 1 à 3, caractérisé en ce qu'on met en oeuvre, par mole de WCl₆, de 2 à 4 équivalents de réduction.

5. Procédé selon l'une quelconque des revendications 1 à 4, caractérisé en ce qu'on met en oeuvre, par mole du composé de formule III, de 2 à 9 équivalents molaires du réactif métallique de faible valence.

6. Procédé selon l'une quelconque des revendications 1 à 5, caractérisé en ce que le solvant est l'éther diéthylique, le dioxanne, le tétrahydrofuranne, le diméthoxyéthane, l'hexane, le benzène ou le toluène, de préférence le tétrahydrofuranne.

7. Procédé selon l'une quelconque des revendications 1 à 6, caractérisé en ce qu'on prépare le réactif métallique de faible valence dans le domaine de température de -78°C à la température ambiante.

8. Procédé selon l'une quelconque des revendications 1 à 7, caractérisé en ce que la réaction entre le réactif métallique de faible valence et le composé de formule III a lieu dans un domaine de température de -78°C à la température de reflux.

9. Procédé selon l'une quelconque des revendications 1 à 8, caractérisé en ce qu'on ajoute au réactif métallique de faible valence préparé, avant l'addition du composé de formule III, encore de 2 à 9 équivalents d'un iodure métallique, de préférence des iodures de métaux alcalins, en particulier l'iodure de lithium.

10. Procédé selon l'une quelconque des revendications 1 à 9, caractérisé en ce que le groupe protecteur pour la fonction hydroxyle est un groupe acétate, un groupe benzoate, un groupe triméthylsilyle ou un groupe tert.-butyldiméthylsilyle.

11. Procédé selon l'une quelconque des revendications 1 à 10, caractérisé en ce qu'on amène à réagir un composé de formule III dans laquelle A et B pris ensemble représentent un époxyde répondant à la formule -(O-CH₂)-, avec soit
I.) du KSeCN, soit
I.a.) dans un alcool, en particulier dans du méthanol, soit
I.b.) dans un éther, en particulier l'éther diéthylique, le dioxanne, le tétrahydrofuranne, le diméthoxyéthane, en présence d'un éther-couronne, en particulier l'éther 18-couronne-6, soit
II.) du (R⁴O)₂P(O)-TeLi dans un éther mentionné ci-dessus ou encore dans du R⁴OH ou R⁴ représente un groupe alkyle, en particulier un groupe éthyle ou un groupe méthyle,
à l'exclusion d'eau et sous l'atmosphère d'un gaz inerte, de préférence au reflux, on traite le produit réactionnel ainsi obtenu et on obtient le produit de formule III dans laquelle A et B pris ensemble forment une liaison double exocyclique répondant à la formule =CH₂.

12. Procédé selon la revendication 11, caractérisé en ce qu'on met en oeuvre, par mole du composé de formule III dans laquelle A et B pris ensemble représentent un époxyde de formule -(O-CH₂)-, de 1 à 10 équivalents molaires des réactifs mentionnés.

13. Procédé selon l'une quelconque des revendications 1 à 12, caractérisé en ce qu'on amène à réagir un composé de formule III dans laquelle
R¹ représente un groupe protecteur pour la fonction hydroxyle auquel R¹ est lié,
R² représente un atome d'hydrogène,
R³ est tel que défini à la revendication 1,
A et B pris ensemble représentent une liaison double exocyclique répondant à la formule =CH₂,
avec du dioxyde de sélénium et un agent de cooxydation, de préférence l'hydroperoxyde de tert.-butyle ou le N-oxyde de pyridinium, dans un solvant, par exemple dans un éther tel que par exemple le dioxanne, le tétrahydrofuranne, le diméthoxyéthane ou dans un hydrocarbure tel que par exemple le benzène, le toluène, à une température supérieure à 50°C, on traite le produit réactionnel ainsi obtenu et on obtient le produit de formule III dans laquelle R¹, R³, ainsi que A et B sont tels que définis ci-dessus et R² représente un groupe hydroxyle, et on protège le cas échéant ce groupe hydroxyle.

14. 5,6:7,8-diépoxy-9,10-sécocholesta-10(19)-ènes répondant à la formule IV 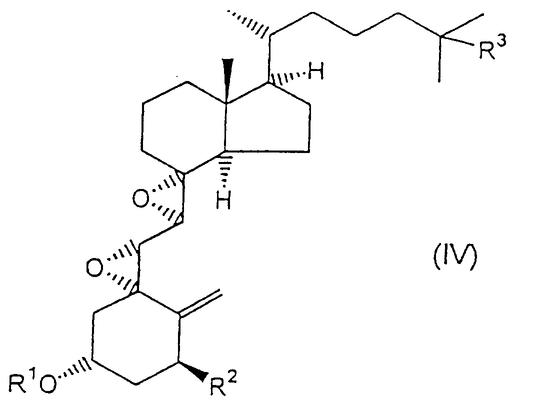 dans laquelle
R¹ représente un atome d'hydrogène ou un groupe protecteur pour la fonction hydroxyle auquel R¹ est lié,
R² représente un atome d'hydrogène ou un groupe OR¹ où R¹ est tel que défini ci-dessus,
R³ représente un atome d'hydrogène ou un groupe OR¹ où R¹ est tel que défini ci-dessus,
les groupes R¹, R² et R³ pouvant être sélectionnés indépendamment l'un de l'autre; toutefois, R² et R³ ne peuvent représenter simultanément un atome d'hydrogène.

15. Procédé pour la préparation des 5,6:7,8:10(19)-triépoxy-9,10-sécocholesta-24-ènes répondant à la formule V 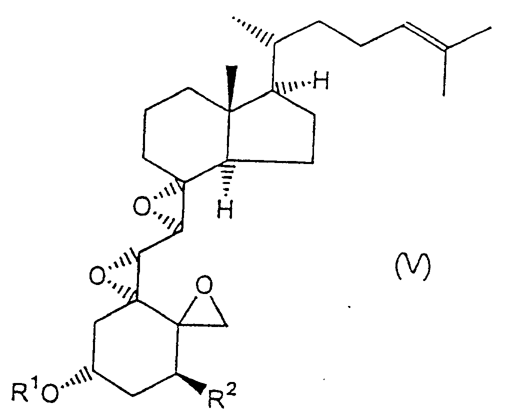 dans laquelle
R¹ représente un atome d'hydrogène ou un groupe protecteur pour la fonction hydroxyle auquel R¹ est lié,
R² représente un atome d'hydrogène ou un groupe OR¹ où R¹ est tel que défini ci-dessus,
les groupes R¹ et R² pouvant être sélectionnés indépendamment l'un de l'autre,
caractérisé en ce qu'on amène à réagir un composé répondant à la formule VI 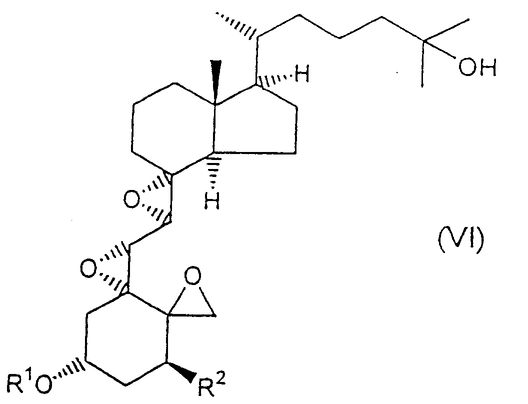 dans laquelle
R¹ et R² sont tels que définis ci-dessus, mais dans laquelle R¹ ne peut représenter un atome d'hydrogène,
avec de l'oxychlorure de phosphore dans de la pyridine, à une température de 0°C à 30°C, en l'absence d'eau, on traite le produit réactionnel ainsi obtenu en éliminant ou non les groupes protecteurs R¹ et on obtient le produit de formule V.

16. 5,6:7,8:10(19)-triépoxy-9,10-sécocholesta-24-ènes répondant à la formule V 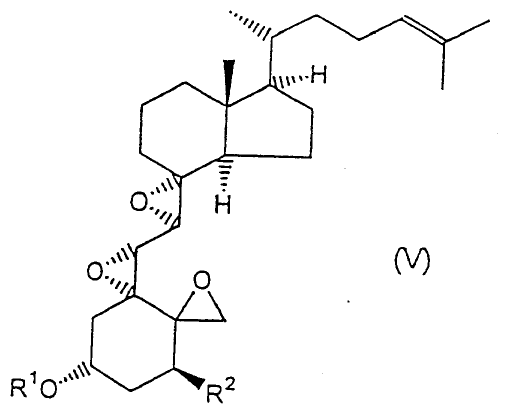 dans laquelle
R¹ représente un atome d'hydrogène ou un groupe protecteur pour la fonction hydroxyle auquel R¹ est lié,
R² représente un atome d'hydrogène ou un groupe OR¹ où R¹ est tel que défini ci-dessus,
les groupes R¹ et R² peuvent être sélectionnés indépendamment l'un de l'autre.
